# EUROPÄISCHE PATENTSCHRIFT

(11) **EP 0 748 633 B1**
(45) Veröffentlichungstag und Bekanntmachung des Hinweises auf die Patenterteilung: **03.09.2003**
(21) Anmeldenummer: 96107785.6
(22) Anmeldetag: 15.05.1996
(51) Int. Cl.: A61L 24/00

(54) **Gewebeklebstoff auf der Basis von Fibrinogen und Verwendung desselben zur Herstellung eines Hämostyptikum**
Adhesive tissue based on fibrinogen and its use for the manufacture of a haemostyptic
Colle pour tissus à base de fibrinogène et son utilisation pour la préparation d'un hémostatique

(30) Priorität: 12.06.1995 DE 19521324
(43) Veröffentlichungstag der Anmeldung: 18.12.1996
(73) Patentinhaber: Baxter Aktiengesellschaft, 1221 Wien (AT)
(72) Erfinder: Seelich, Thomas, Dr., 1030 Wien (AT); Turecek, Peter, Dr., 3400 Klosterneuburg, Weidling (AT)
(74) Vertreter: Kolb, Helga, Dr. Dipl.-Chem.

(56) Entgegenhaltungen:
- AT-B- 369 990
- DATABASE BIOSIS [Online] BIOSCIENCES INFORMATION SERVICE, PHILADELPHIA, PA, US SUZUKI M ET AL.: "CLINICAL APPLICATION OF THE FIBRIN ADHESIVE" XP002040697 & OTOLARYNGOLOGY, Bd. 56, Nr. 11, 1984, Seiten 949-953, TOKYO

## Beschreibung

Die Erfindung betrifft einen Gewebeklebstoff und die Verwendung desselben zur Herstellung eines Hämostyptikums, insbesondere betrifft die Erfindung ein lagerstabiles, pharmazeutisches Präparat zur Blutstillung, Verklebung von verletztem Gewebe und zur Förderung der Wundheilung auf Basis von Fibrinogen und einem Blutgerinnungsfaktor-Aktivator.

Gewebeklebstoffe auf Basis von Fibrinogen werden zum nahtlosen bzw. naht-unterstützenden Verbinden von menschlichen oder tierischen Gewebe- bzw. Organteilen, zur Wundversiegelung, Blutstillung und Förderung der Wundheilung eingesetzt.

Ihre Wirkungsweise beruht darauf, dass durch Einwirkung von Thrombin das im gebrauchsfertigen, flüssigen Gewebeklebstoff enthaltene (lösliche) Fibrinogen in (unlösliches) Fibrin übergeführt und der im Gewebeklebstoff enthaltene Faktor XIII zu Faktor XIIIa aktiviert wird. Dieser vernetzt das gebildete Fibrin zu einem Hochpolymer, was für die Wirksamkeit des Gewebeklebstoffes wesentlich ist. Die benötigte Thrombinaktivität kann entweder aus dem zu klebenden Gewebe (den Wundflächen) selbst stammen oder in Form einer Thrombin- und Ca²⁺-Ionen-enthaltenden Lösung dem Gewebeklebstoff bei der Klebung zugesetzt werden.

Zur Blutstillung bzw. Gewebeklebung sind die folgenden Mittel bekannt, die auf der Ausnutzung der letzten Phase der Blutgerinnung (Umwandlung von Fibrinogen in Fibrin, und Vernetzung des Fibrins durch Faktor XIIIa) beruhen:
a) Thrombin allein (als Pulver, Lösung oder in Kombination mit einem saugfähigen Träger, wie z.B. Collagenvlies): Ein solches Mittel löst die Gerinnung des im Patientenblut vorhandenen Fibrinogens aus. Die Festigkeit des entstehenden Gerinnsels hängt jedoch wesentlich von der Fibrinogenkonzentration ab, die in diesem Fall relativ niedrig ist (ca. 3 mg/ml). Die blutstillende bzw. klebende Wirkung von solchen Präparaten ist daher relativ gering bzw. kann im Falle einer Hypofibrinogenämie sogar völlig fehlen.
b) Gewebeklebstoffe auf Basis von Fibrinogen und Faktor XIII, entsprechend AT-B-359 653, AT-B-359 652 und AT-B-369 990. Solche Präparate weisen einen wesentlich höheren Gehalt an Fibrinogen und Faktor XIII auf als menschliches Blut; nach Einwirkung von Thrombin (in Gegenwart von Ca²⁺-Ionen) ergeben solche Präparate Clots von hoher Festigkeit und guter Haftfähigkeit am Gewebe, so dass mit solchen Präparaten an sich eine gute blutstillende und klebende Wirkung erreicht wird.
   Diese Präparate werden im allgemeinen in Kombination mit einer Thrombinlösung angewendet, wobei die Fibrinogen enthaltende Lösung unmittelbar vor der Anwendung möglichst rasch und vollkommen mit der Thrombinlösung gemischt werden muss, damit eine optimale Wirkung erzielt wird. Zur einfachen Anwendung derartiger Gewebeklebstoffe wurde daher auch ein eigenes Misch- und Applikationsgerät (Duploject®, Immuno AG) entwickelt.
   Dennoch besteht ärztlicherseits der Wunsch nach einfacher anzuwendenden, gut wirksamen Präparaten zur Blutstillung und Gewebeklebung.
c) Trockenpräparate in Form saugfähiger Vliese, die Fibrinogen und Thrombin bzw. in Gegenwart von Körperflüssigkeit Thrombin freisetzende Substanzen enthalten (DE 31 05 624 und DE 32 14 337).
   Diese Präparate müssen vollkommen trocken gelagert werden, um eine vorzeitige Aktivierung der Blutgerinnung durch Einwirkung von Thrombin auf Fibrinogen , die bereits in Gegenwart von Spuren Feuchtigkeit im Präparat einsetzt, zu vermeiden. Solche Präparate sind daher üblicherweise in Kombination mit einem Trockenmittel luft- bzw. feuchtigkeitsdicht verpackt. Die notwendige Trockenheit solcher Präparate bewirkt aber auch eine unerwünschte Sprödigkeit, die das Aufbringen auf Wundflächen erschwert.
   Ein weiteres grundsätzliches Problem solcher Präparate besteht darin, dass sich bei der Anwendung, d.h. nach Befeuchten mit Blut oder Wundsekret, das Thrombin aufgrund seiner besseren Löslichkeit auf jeden Fall wesentlich schneller löst als das gleichzeitig vorhandene Fibrinogen. Fibrinogen kann sich aber in einem thrombinhaltigen Medium überhaupt nicht mehr wie erwünscht lösen: Bei beginnender Auflösung eines Fibrinogenpartikels entsteht an dessen Oberfläche sofort eine feste Fibrinschicht, die die weitere Auflösung des Fibrinogenpartikels hemmt. Von der in einem solchen Präparat vorhandenen Gesamtmenge an Fibrinogen kann also bestenfalls nur ein kleiner Bruchteil wirksam werden.
d) Schliesslich ist noch ein sogenannter Einkomponentenkleber vorgeschlagen worden (EP-253 198), dessen Anwendung gegenüber Gewebeklebern entsprechend AT-359 653 und AT-359 652 vereinfacht sein soll. Entsprechend diesem Vorschlag enthalten solche Einkomponentenkleber in der Anwendungslösung Fibrinogen, Faktor XIII, einen Thrombininhibitor, einen erhöhten Gehalt an Prothrombin und Prothrombinkomplexfaktoren und Calciumionen.

Derartige Präparate sind in lyophilisierter Form stabil. Die Auflösung vor der Anwendung soll mit einer verdünnten CaCl₂-Lösung erfolgen, wobei dann das gelöste Präparat bei der Anwendung in Abhängigkeit von der Ca²⁺-Konzentration mehr oder weniger rasch gerinnen soll. Der eigentliche Aktivator, der die Gerinnung letztlich auslöst, ist das vorhandene CaCl₂ (anstelle von Thrombin). Dieses muss sehr genau dosiert werden, um einerseits die Auflösung vor Einsetzen der Gerinnung überhaupt zu ermöglichen, und um andererseits auf der Wundfläche dennoch eine genügend rasche Gerinnung zu erreichen.

Die Anwendung derartiger Präparate erscheint daher keineswegs einfacher, sondern sogar eher schwieriger als von bekannten Gewebeklebstoffen entsprechend b). Es überrascht daher nicht, dass sich derartige Präparate nach EP-253 198 bisher in der Praxis nicht durchgesetzt haben.

Aufgabe der vorliegenden Erfindung ist es daher, ein einfach anzuwendendes gut wirksames Präparat zur Blutstillung, Klebung von verletztem Gewebe und zur Förderung der Wundheilung zu schaffen, das die oben diskutierten Nachteile bekannter Präparate nicht aufweist.

Diese Aufgabe wird erfindungsgemäss durch ein Präparat gelöst, das als wirksame Bestandteile Fibrinogen und einen Aktivator oder Pro-Aktivator von (im Blut vorhandenem) Prothrombin enthält, der mit Fibrinogen selbst nicht reagiert. Damit wird das im Patientenblut vorhandene Prothrombin zu Thrombin umgewandelt, welches wiederum die Fibrinclot-Bildung bewirkt.

Der erfindungsgemässe Gewebeklebstoff ist im Hinblick auf die Anwesenheit von Prothrombin abgereichert, so dass der Gehalt weniger als 5 E Prothrombin/g Fibrinogen beträgt, vorzugsweise weniger als 1 E/g, am meisten bevorzugt weniger als die Nachweisgrenze mit üblichen bekannten analytischen Methoden. Als Prothrombinaktivator wird vorzugsweise Faktor Xa, beispielsweise hergestellt gemäss DE 43 25 872, verwendet.

Ein solches Präparat enthält vorteilhafterweise zusätzlich Phospholipide, vorzugsweise assoziiert mit dem Aktivator in vesikulärer Form, aber auch Gerinnungsfaktor XIII, Ca²⁺-Ionen bzw. ein Calcium-Salz, oder einen Inhibitor der Fibrinolyse, der den Aktivator nicht oder nur mässig hemmt. Bevorzugte Fibrinolyseinhibitoren sind Aprotinin oder α₂-Plasmininhibitor.

Faktor XIII kann in einer Menge von mindestens 80 Einheiten/g Fibringen, bevorzugt von mindestens 150 Einheiten/g Fibringen enthalten sein.

Zur Verbesserung der Lagerstabilität kann ein solches Präparat zusätzlich geringe Mengen eines Inhibitors der Gerinnung, z.B. eines Thrombininhibitors, wie AT-III, AT-III-Heparin-Komplex oder Hirudin enthalten.

Das Präparat kann in flüssiger bzw. flüssig-tiefgefrorener Form oder als Lyophilisat, vorzugsweise gebunden an ein biologisch resorbierbares, wasserlösliches oder in Wasser schwer lösliches Trägermaterial, wie Collagenvlies, Fibrinschaum, Gelatine oder Cellulose, gegebenenfalls in modifizierter Form, wie Oxycellulose, vorliegen.

Gleichfalls können auch als Trägermaterialien Polymilchsäure, Chitin, Chitosan, Proteoglykane, Glykosaminglykane, wie Chondroitinsulfate, Hyaluronsäuren oder Keratansulfate, Heparansulfate, Dextrane, Elastin, Fibronectin, Vitronektin, Laminin, Tenascin, Spongiosa-Knochenmaterial, vorzugsweise in entkalkter Form, entsprechend EP-321 442, und/oder Hydroxylapatit verwendet werden.

Die Bestandteile des Gewebeklebstoff-Präparates sind vorzugsweise aus menschlichem Blutplasma gewonnen und zur Inaktivierung möglicherweise vorhandener Viren behandelt. Aber auch das Gewebeklebstoff-Präparat selbst kann entsprechend behandelt werden, beispielsweise durch eine Hitzebehandlung, wie gemäss EP-159 311.

Das Präparat kann zusätzlich weitere Wirkstoffe, wie Antibiotika, Wachstumshormone, weitere Plasmaproteine, wie beispielsweise Fibronectin, sowie weitere Gerinnungsfaktoren, insbesondere Faktor V, (mit Ausnahme von Prothrombin) enthalten.

Eine Aktivierung, d.h. Umwandlung von Fibrinogen in Fibrin, und eine Vernetzung des Fibrins durch Faktor XIIIa erfolgt erst bei Kontakt mit Blut bzw. verletztem Gewebe und zwar im wesentlichen durch das dort vorhandene Prothrombin und gegebenenfalls Faktor XIII. Prothrombin wird durch den im Präparat enthaltenen Prothrombin-Aktivator, vorzugsweise Faktor Xa, in Thrombin umgewandelt. Thrombin löst sodann den eigentlichen Gerinnungsvorgang aus, d.h. das im Präparat ebenfalls vorhandene Fibrinogen sowie das Fibrinogen des Blutes selbst wird in Fibrin umgewandelt; Faktor XIII wird durch Thrombin zu Faktor XIIIa aktiviert. Dieser vernetzt das gebildete Fibrin zu einem Hochpolymer, wodurch die Festigkeit des gebildeten Fibringerinnsels wesentlich erhöht wird. Auf der letzteren Reaktion beruht im wesentlichen die blutstillende und klebende Wirkung des Präparates.

Die bevorzugte Menge an Aktivator liegt im Bereich von 0,001 bis 10 E Faktor Xa oder deren Äquivalente, vorzugsweise 0,1 bis 1 Einheit pro cm³ Gewebeklebstoff. Im Falle eines Vlieses ist es bevorzugt, soviel Prothrombin-Aktivator oder -Proaktivator pro cm² zu verwenden, dass 5 bis 500 µg, vorzugsweise 10 bis 100 µg Prothrombin in weniger als 60 sec, vorzugsweise in weniger als 30 sec aktiviert werden.

Ein besonderes Merkmal des erfindungsgemässen Präparates ist seine Stabilität, und zwar nicht nur in trockenem Zustand, sondern auch in Gegenwart von Wasser bzw. als Lösung. Fibrinogen wird erst bei der Anwendung, d.h. beim Kontakt mit dem Blut bzw. Wundsekret des Patienten aktiviert, wobei eine überraschend gute blutstillende und klebende Wirkung erzielt wird.

Anstelle von Faktor Xa können auch andere Prothrombin-Aktivatoren oder -Proaktivatoren, die mit Fibrinogen nicht oder nur sehr langsam reagieren, verwendet werden, wie z.B. aktivierte Gerinnungsfaktoren ausser Thrombin, darunter die Faktoren VIIa, IXa, XIa und XIIa, aber auch "Tissue-Faktor". Gleichfalls können auch geeignete Aktivatoren aus geeigneten Schlangengiften oder Bakterien, die beispielsweise in Thromb. Haemostas. 65, 627-630 (1991) beschrieben sind, verwendet werden.

Das Präparat kann aber auch als sogenannter 'Trockenkleber' in fester Form, vorzugsweise als dünnes Vlies oder Pulver, vorliegen. Der Restwassergehalt in diesem Präparat ist nicht kritisch. Vorzugsweise beträgt der Restwassergehalt weniger als 10%, am meisten bevorzugt weniger als 5%. Ein solches Präparat liegt vorzugsweise als dünnes Vlies mit nur wenig oder keinem Gehalt an wasserunlöslichem Trägermaterial, wie Collagen oder Fibrin, vor. Zur Verklebung von zwei (weichen) Gewebeteilen wird ein passendes Stück des 'Trockenklebers' auf eine Wundfläche aufgebracht und anschliessend die zweite Wundfläche (der zweite Gewebeteil) adaptiert und kurz angedrückt. Durch das vorhandene Blut bzw. Wundsekret löst sich das Präparat rasch auf und verfestigt sich anschliessend mit dem Einsetzen der Gerinnung, wodurch die klebende und blutstillende Wirkung zustande kommt. Durch das erfindungsgemässe 'Trockenmaterial' wird somit ein fester, beidseitig klebender Gewebeklebstoff zur Verfügung gestellt, der sich vor allem zur Klebung von Weichteilen, wie Leber oder Milz, eignet.

### Beispiel 1

### Collagen-Vlies mit Fibrinogen, Faktor XIII, Faktor Xa und Phospholipiden (als Phospholipid-Faktor Xa-Komplex)

### 1.1 Herstellung

Ein käuflich erhältliches Collagen-Vlies (Tissue Vlies, Immuno, ca. 5 mm dick) wurde in Stücke von ca. 2 x 2 cm geschnitten und die Stücke in einer Lösung folgender Zusammensetzung getränkt, tiefgefroren und lyophilisiert:

| | | |
|---|---|---|
| Fibrinogen | 10 mg/ml | |
| Faktor XIII | 3,5 E/ml | |
| Faktor Xa | 0,13 E/ml | Phospholipid-Faktor |
| PCPS* | 0,0025 ml/mg | Xa-Komplex |
| Humanalbumin | 1,5 g/l | |
| Ca²⁺ | 5 mmol/l | |
| NaCl | 180 mmol/l | |
| Tris·HCl | 20 mmol/l | |
| Saccharose | 50 mg/l | |
| pH | 7,3 | |

| | | |
|---|---|---|
| * Phosphatidylcholin + Phosphatidylserin | | |

Die in der Lösung enthaltenen Wirkstoffe wurden auf folgende Weise hergestellt:

### Fibrinogen:

Ein aus humanem Citratplasma erhaltenes Kryopräzipitat wurde mit der 10-fachen Menge einer Pufferlösung, enthaltend pro l 6 g Natriumcitrat·2H₂O, 7 g NaCl, 13 g ε-Aminocapronsäure und 600 IE Heparin, gelöst und der pH-Wert auf 7,3 eingestellt. Sodann wurden unter Rühren pro l Lösung 150 g festes Glycin zugegeben und der pH-Wert auf 7,3 nachgestellt.

Der gebildete Proteinniederschlag wurde durch Zentrifugation abgetrennt, nochmals w.o. gelöst und die Fällung mit Glycin wie oben wiederholt. Der nach Zentrifugation erhaltene Niederschlag wurde bei 0-2°C mit einer weiteren Pufferlösung (pH 6,5), enthaltend pro 1 6,6 g Natriumcitrat 2H₂O, 3,4 g NaCl und 200 IE Heparin gewaschen, die Proteinkonzentration auf 40 g/l und der pH auf 7,3 eingestellt, gereinigtes Humanalbumin (6 g/l) zugesetzt, und die Lösung tiefgefroren und lyophilisiert.

Zur Inaktivierung möglicherweise vorhandener Viren wurde das Material bei einer Restfeuchte von 7,5 Gew.-% 10 Stunden auf 60°C, und 1 Stunde auf 80°C erhitzt. Das so erhaltene gereinigte lyophilisierte Fibrinogenpräparat enthielt ausser dem zugesetzten Humanalbumin nur mehr Spuren anderer Plasmaproteine. Prothrombin war nur noch in einer Menge von 4 E/g Fibrinogen enthalten

Die Bestimmung der Proteinzusammensetzung erfolgte mittels SDS-Polyacrylamidgelelektrophorese einer nicht reduzierten und einer reduzierten Probe des Präparates, Färbung mit Coomassie-Blau und densitometrischer Auswertung.

Die Bestimmung des Prothrombingehaltes erfolgte nach der Einstufenmethode, basierend auf der Prothrombinzeit nach Quick, Journal Biological Chemistry 109, pp 73 (1935).

Die Herstellung und Testung eines gereinigten virusinaktivierten Faktor XIII-Konzentrates erfolgte entsprechend Beispiel 3C des europäischen Patents EP-0637451, auf welches hiermit bezug genommen wird.

Faktor Xa wurde wie in DE-43 25 872 beschrieben hergestellt und getestet.

Die Herstellung des Phospholipid (PCPS)-Faktor Xa-Komplexes erfolgte durch Herstellung von PCPS-Vesikeln nach dem Extrusionsverfahren von Olson et al, Biophysica et Biochimica Acta 557, pp 9-23, 1979, und anschliessender Komplexierung mit dem Faktor Xa. In einem 100 ml-Kolben wurden 40,0 mg 1,2-Dioleoyl-sn-glycero-3-phosphocholin und 10,0 mg 1-Palmitoyl-2-oleoyl-sn-glycero-3-phosphoserin (Avanti Polar Lipids) in 5 ml Chloroform gelöst und mit Hilfe eines Rotationsverdampfers unter reduziertem Druck und einer Temperatur von 30°C eingeengt. Nach vollständiger Entfernung des Lösungsmittels wurde noch über 30 min bei 30 mbar im Vakuum gehalten. Der Phospholipid-Film wurde anschliessend durch Zugabe von 5 ml TBS-Puffer (20 mM Tris, 150 mM NaCl, pH 7,4 ) und leichtem Schütteln über eine Stunde bei Raumtemperatur hydratisiert und anschliessend lyophilisiert. Nach Zugabe von 5 ml Wasser wurde die daraus resultierende Dispersion multilammellarer Vesikel 10mal durch zwei übereinander gelegte 100 nm Polycarbonatfilter mit N₂-Druck gepresst (10 ml-Thermobarrel-Extruder, Lipex-Biomembranes Inc, Vancouver, Kanada). Die Bestimmung der Partikelgrösse mit Hilfe der dynamischen Lichtstreuung ergab einen mittleren Durchmesser von ca. 100 nm. Der so hergestellten Vesikel-Dispersion wurde Faktor Xa-(TBS-Puffer) zugemischt.

Anschliessend wurde eine CaCl₂-Konzentration von 5 mM sowie eine Saccharose-Konzentration von 5% (w/v) eingestellt und lyophilisiert. Ein auf diese Weise hergestelltes Lyophilisat wies nach Rekonstitution mit dem entsprechenden Volumen H₂O eine Phospholipid-Konzentration von 2,9 mg/ml sowie eine Faktor Xa-Konzentration von 79 E/ml auf.

### 1.2 Wirksamkeit

Die Wirksamkeit, d.h. die klebende und blutstillende Wirkung der nach 1.1 hergestellten Präparate wurde in einem Nierenpolresektionsmodell am Kaninchen getestet. Dabei wird dem Versuchstier unter Narkose mit Kedamin und Xylazin (Nachdosierung Pentobarbital) eine Niere freigelegt und mit dem Skalpell der Nierenpol entfernt. Es entsteht eine etwa kreisrunde, gleichmässig stark blutende Fläche von ca. 1-1,5 cm Durchmesser. Das Testpräparat wurde auf die blutende Fläche aufgelegt und genau 30 sec mit dem Finger leicht angedrückt, danach losgelassen. Die Haftung des Präparates wird beobachtet und die Zeit bis zum voilständigen Stillstand der Blutung gemessen.

### Ergebnisse:

Das nach 1.1 hergestellte Präparat haftete gut auf der stark blutenden Fläche. Ein vollständiger Stillstand der Blutung wurde nach 1 Minute erreicht.

## Patentansprüche

1. Stabiler Gewebeklebstoff auf der Basis von Fibrinogen, **dadurch gekennzeichnet, dass** dieser einen Aktivator oder Pro-Aktivator von im Blut vorhandenem Prothrombin, der mit Fibrinogen selbst nicht reagiert, enthält, wobei der Gewebeklebstoff weniger als 5 Einheiten Prothrombin /g Fibrinogen umfasst.

2. Gewebeklebstoff nach Anspruch 1, **dadurch gekennzeichnet, dass** dieser weniger als eine Einheit Prothrombin / g Fibrinogen umfasst.

3. Gewebeklebstoff nach Anspruch 1 und 2, **dadurch gekennzeichnet, dass** als Aktivator Faktor Xa enthalten ist.

4. Gewebeklebstoff nach Anspruch 3, **dadurch gekennzeichnet, dass** pro cm³ Gewebeklebstoff 0,001 bis 10 Einheiten Faktor Xa oder deren Äquivalente, vorzugsweise 0,1 bis 1 Einheit, enthalten sind.

5. Gewebeklebstoff nach Anspruch 1 bis 4, **dadurch gekennzeichnet, dass** zusätzlich ein Inhibitor der Gerinnung oder der Fibrinolyse enthalten ist.

6. Gewebeklebstoff nach Anspruch 5, **dadurch gekennzeichnet, dass** der Inhibitor der Gerinnung Antithrombin III oder Antithrombin III-Heparin-Komplex oder Hirudin ist.

7. Gewebeklebstoff nach Anspruch 5, **dadurch gekennzeichnet, dass** der Inhibitor der Fibrinolyse Aprotinin oder Alpha-2-Antiplasmin ist.

8. Gewebeklebstoff nach Anspruch 1 bis 7, **dadurch gekennzeichnet, dass** zusätzlich Phospholipide enthalten sind.

9. Gewebeklebstoff nach Anspruch 8, **dadurch gekennzeichnet, dass** die Phospholipide mit dem Aktivator in vesikulärer Form assoziiert sind.

10. Gewebeklebstoff nach einem oder mehreren der Ansprüche 1 bis 9, **dadurch gekennzeichnet, dass** dieser zusätzlich Faktor V enthält.

11. Gewebeklebstoff nach einem oder mehreren der Ansprüche 1 bis 10, **dadurch gekennzeichnet, dass** dieser zusätzlich Faktor XIII enthält.

12. Gewebeklebstoff nach Anspruch 11, **dadurch gekennzeichnet, dass** Faktor XIII in einer Menge von mindestens 80 Einheiten/g Fibrinogen enthalten ist.

13. Gewebeklebstoff nach Anspruch 11, **dadurch gekennzeichnet, dass** Faktor XIII in einer Menge von mindestens 150 Einheiten/g Fibrinogen enthalten ist.

14. Gewebeklebstoff nach einem oder mehreren der Ansprüche 1 bis 13, **dadurch gekennzeichnet, dass** die Bestandteile des Gewebeklebstoffes oder der Gewebeklebstoff selbst zur Inaktivierung von Viren vorbehandelt ist (sind).

15. Gewebeklebstoff nach einem oder mehreren der Ansprüche 1 bis 14, **dadurch gekennzeichnet, dass** dieser zusätzlich Fibronektin und gegebenenfalls einen Fibrinolyse-Inhibitor enthält.

16. Gewebeklebstoff nach einem oder mehreren der Ansprüche 1 bis 15, **dadurch gekennzeichnet, dass** dieser als weitere Wirkstoffe zusätzlich Antibiotika, Wachstumshormone und/oder von Prothrombin unterschiedliche Gerinnungsfaktoren enthält.

17. Gewebeklebstoff nach einem oder mehreren der Ansprüche 1 bis 16, **dadurch gekennzeichnet, dass** dieser als flüssiges Präparat vorliegt.

18. Gewebeklebstoff nach Anspruch 17, **dadurch gekennzeichnet, dass** dieser in flüssig-tiefgefrorener Form vorliegt.

19. Gewebeklebstoff nach einem oder mehreren der Ansprüche 1 bis 16, **dadurch gekennzeichnet, dass** dieser in fester oder lyophilisierter Form, vorzugsweise als Trockenkleber vorliegt.

20. Gewebeklebstoff nach einem oder mehreren der Ansprüche 1 bis 19, **dadurch gekennzeichnet, dass** dieser auf einem biologisch resorbierbaren, wasserunlöslichen oder in Wasser schwer löslichen Trägermaterial aufgebracht ist.

21. Gewebeklebstoff nach Anspruch 20, **dadurch gekennzeichnet, dass** als Trägermaterial lies, Collagenschaum, Fibrin, Gelatine oder Cellulose, gegebenenfalls in modifizierter Form, wie Oxycellulose, verwendet werden.

22. Gewebeklebstoff nach Anspruch 21, **dadurch gekennzeichnet, dass** pro cm² Vlies soviel Prothrombinaktivator enthalten ist, um 5 bis 500 µg, vorzugsweise 10 bis 100 µg, Prothrombin in weniger als 60 sec, vorzugsweise in weniger als 30 sec, zu aktivieren.

23. Verwendung des Gewebeklebstoffes gemäss einem oder mehreren der Ansprüche 1 bis 22 zur Herstellung eines Medikamentes als Hämostyptikum.

## Claims

1. Stable tissue adhesive based on fibrinogen, **characterised in that** the latter contains an activator or pro-activator of prothrombin present in the blood, which does not react with fibrinogen itself, wherein the tissue adhesive comprises less than 5 units of prothrombin/g of fibrinogen.

2. Tissue adhesive according to claim 1, **characterised in that** the latter comprises less than one unit of prothrombin/g of fibrinogen.

3. Tissue adhesive according to claim 1 and 2, **characterised in that** factor Xa is present as activator.

4. Tissue adhesive according to claim 3, **characterised in that** 0.001 to 10 units of factor Xa or equivalents thereof, preferably 0.1 to 1 unit, are present per cm³ of tissue adhesive.

5. Tissue adhesive according to claim 1 to 4, **characterised in that** an inhibitor of coagulation or of fibrinolysis is additionally present.

6. Tissue adhesive according to claim 5, **characterised in that** the inhibitor of coagulation is antithrombin III or antithrombin III-heparin complex or hirudin.

7. Tissue adhesive according to claim 5, **characterised in that** the inhibitor of fibrinolysis is aprotinin or alpha-2-antiplasmin.

8. Tissue adhesive according to claim 1 to 7, **characterised in that** phospholipids are additionally present.

9. Tissue adhesive according to claim 8, **characterised in that** the phospholipids are associated with the activator in vesicular form.

10. Tissue adhesive according to one or more of claims 1 to 9, **characterised in that** the latter additionally contains factor V.

11. Tissue adhesive according to one or more of claims 1 to 10, **characterised in that** the latter additionally contains factor XIII.

12. Tissue adhesive according to claim 11, **characterised in that** factor XIII is present in a quantity of at least 80 units/g of fibrinogen.

13. Tissue adhesive according to claim 11, **characterised in that** factor XIII is present in a quantity of at least 150 units/g of fibrinogen.

14. Tissue adhesive according to one or more of claims 1 to 13, **characterised in that** the constituents of the tissue adhesive or the tissue adhesive itself is (are) pre-treated to inactivate viruses.

15. Tissue adhesive according to one or more of claims 1 to 14, **characterised in that** the latter additionally contains fibronectin and optionally a fibrinolysis inhibitor.

16. Tissue adhesive according to one or more of claims I to 15, **characterised in that** the latter additionally contains antibiotics, growth hormones and/or coagulation factors different to prothrombin as further active ingredients.

17. Tissue adhesive according to one or more of claims 1 to 16, **characterised in that** the latter is present as a liquid preparation.

18. Tissue adhesive according to claim 17, **characterised in that** the latter is present in liquid deep-frozen form.

19. Tissue adhesive according to one or more of claims 1 to 16, **characterised in that** the latter is present in solid or lyophilised form, preferably as dry adhesive.

20. Tissue adhesive according to one or more of claims I to 19, **characterised in that** the latter is applied to a biologically resorbable, water-insoluble carrier material or one which is difficult to dissolve in water.

21. Tissue adhesive according to claim 20, **characterised in that** nonwoven, collagen foam, fibrin, gelatine or cellulose, optionally in modified form, such as oxycellulose, are used as carrier material.

22. Tissue adhesive according to claim 21, **characterised in that** per cm² of nonwoven, as much prothrombin activator is present in order to activate 5 to 500 µg, preferably 10 to 100 µg, of prothrombin in less than 60 seconds, preferably in less than 30 seconds.

23. Use of the tissue adhesive according to one or more of claims 1 to 22 for producing a medicament as haemostyptic.

## Revendications

1. Colle pour tissus stable à base de fibrinogène, **caractérisée en ce que** celle-ci contient un activateur ou un pro-activateur de la prothrombine présente dans le sang, ne résistant pas avec le fibrinogène lui-même, la colle pour tissus comprenant moins de 5 unités de prothrombine/g de fibrinogène.

2. Collé pour tissus selon la revendication 1, **caractérisée en ce que** celle-ci comprend moins d'une unité de prothrombine/g fibrinogène.

3. Colle pour tissus selon la revendication 1 ou 2, **caractérisée en ce qu'**un facteur Xa est contenu en tant qu'activateur.

4. Colle pour tissus selon la revendication 3, **caractérisée en ce que** de 0,001 à 10 unités de facteur Xa ou bien de ses équivalents, de préférence de 0,1 à 1 unité, sont contenus par cm' de colle pour tissus.

5. Colle pour tissus selon la revendication 1 à 4, **caractérisée en ce qu'**en plus un inhibiteur de coagulation ou bien de la fibrinolyse est contenu.

6. Colle pour tissus selon la revendication 5, **caractérisée en ce que** l'inhibiteur de la coagulation est l'antithrombine III ou un complexe antithrombine III-héparine ou bien l'hiridine.

7. Colle pour tissus selon la revendication 5, **caractérisée en ce que** l'inhibiteur de la fibrinolyse est l'aprotinine ou l'alpha-2-antiplasmine.

8. Colle pour tissus selon les revendications 1 à 7, **caractérisée en ce qu'**en plus sont contenus des phospholipides.

9. Colle pour tissus selon la revendication 8, **caractérisée en ce que** les phospholipides sont associés à l'activateur, sous une forme vésiculaire.

10. Colle pour tissus selon une ou plusieurs des revendications 1 à 9, **caractérisée en ce que** celle-ci contient en plus un facteur V.

11. Colle pour tissus selon une ou plusieurs des revendications 1 à 10, **caractérisée en ce que** celle-ci comprend en plus un facteur XIII.

12. Colle pour tissus selon la revendication 11, **caractérisée en ce que** le facteur XIII est contenu en une quantité d'au moins 80 unités/g de fibrinogène.

13. Colle pour tissus selon la revendication 11, **caractérisée en ce que** le facteur XIII est contenu en une quantité d'au moins 150 unités/g de fibrinogène.

14. Colle pour tissus selon une ou plusieurs des revendications 1 à 13, **caractérisée en ce que** les composants de la colle pour tissus ou la colle pour tissus elle-même est (sont) prétraité(s) pour inactiver des virus.

15. Colle pour tissus selon une ou plusieurs des revendications 1 à 14, **caractérisée en ce que** celle-ci comprend en plus de la fibronectine et, le cas échéant, un inhibiteur de fibrinolyse.

16. Colle pour tissus selon une ou plusieurs des revendications 1 à 15, **caractérisée en ce que** celle-ci contient, à titre d'autres principes actifs, en plus des antibiotiques, des hormones de croissance et/ou des facteurs de coagulation différents de la prothrombine.

17. Colle pour tissus selon une ou plusieurs des revendications 1 à 16, **caractérisée en ce que** celle-ci se présente sous la forme de préparation liquide.

18. Colle pour tissus selon la revendication 17, **caractérisée en ce que** celle-ci se présente sous une forme liquide portée à une basse température de congélation.

19. Colle pour tissus selon une ou plusieurs des revendications 1 à 16, **caractérisée en ce que** celle-ci se présente sous une forme solide ou lyophilisée, de préférence sous la forme d'adhésif à sec.

20. Colle pour tissus selon une ou plusieurs des revendications 1 à 19, **caractérisée en ce que** celle-ci est appliquée sur un matériau support résorbable biologiquement, insoluble dans l'eau, ou difficilement soluble dans l'eau.

21. Colle pour tissus selon la revendication 20, **caractérisée en ce que** l'on utilise comme matériau support un matelas de fibre, une mousse de collagène, de la fibrine, de la gélatine ou de la cellulose, le cas échéant sous forme modifiée, tel que de l'oxycellulose.

22. Colle pour tissus selon la revendication 21, **caractérisée en ce que**, par cm² de matelas de fibre, est contenue une quantité d'activateur de prothrombine, permettant d'activer de 5 à 500 µg, de préférence de 10 à 100 µg de prothrombine en moins de 60 secondes, de préférence en moins de 30 secondes.

23. Utilisation de la colle pour tissus selon une ou plusieurs des revendications 1 à 22 pour la préparation d'un médicament servant d'hémostyptique.
